(19) Europäisches Patentamt

European Patent Office

Office européen des brevets

(11) **EP 1 458 699 B1**

(12) **EUROPEAN PATENT SPECIFICATION**

(45) Date of publication and mention
of the grant of the patent:
**09.11.2005 Bulletin 2005/45**

(51) Int Cl.⁷: **C07D 301/10**

(86) International application number:
**PCT/US2002/037026**

(21) Application number: **02803666.3**

(22) Date of filing: **19.11.2002**

(87) International publication number:
**WO 2003/044003 (30.05.2003 Gazette 2003/22)**

(54) **A PROCESS AND SYSTEMS FOR THE EPOXIDATION OF AN OLEFIN**

VERFAHREN UND SYSTEME ZUR EPOXIDIERUNG EINES OLEFINS

PROCEDE ET SYSTEMES D'EPOXYDATION D'OLEFINE

(84) Designated Contracting States:
**AT BE BG CH CY CZ DE DK EE ES FI FR GB GR
IE IT LI LU MC NL PT SE SK TR**

(30) Priority: **20.11.2001 US 331874 P
20.11.2001 US 331828 P**

(43) Date of publication of application:
**22.09.2004 Bulletin 2004/39**

(73) Proprietor: **SHELL INTERNATIONALE
RESEARCH MAATSCHAPPIJ B.V.
2596 HR Den Haag (NL)**

(72) Inventors:
• **CHIPMAN, Peter, Ingraham
Houston, TX 77041 (US)**
• **KOBE, Jeffrey, Michael
Katy, TX 77450 (US)**
• **TE RAA, Arend, Jan
NL- Dordrecht (NL)**

• **VANDERWILP, Brian, Scott
Katy, TX 77450 (US)**
• **RUBINSTEIN, Leonid, Isaakovich
Houston, TX 77006 (US)**
• **WERMINK, Thijs
2253 RD Voorschoten (NL)**

(74) Representative: **Zeestraten, Albertus W. J.
Shell International B.V.,
Intellectual Property Services,
P.O. Box 384
2501 CJ The Hague (NL)**

(56) References cited:
**EP-A- 0 352 850          WO-A-98/58920**

• **SERAFIN, J.G., LIU, A.C., SEYEDMONIR, S.R.:
"Surface science and the silver-catalyzed
epoxidation of ethylene: an industrial
perspective" JOURNAL OF MOLECULAR
CATALYSIS A : CHEMICAL, vol. 131, 1998, pages
157-168, XP002230219**

EP 1 458 699 B1

## Description

FIELD OF THE INVENTION

The invention relates to a process for the epoxidation of an olefin, which process comprises reacting a feed comprising the olefin, oxygen and a reaction modifier in the presence of a highly selective silver-based catalyst. The invention also relates to systems suitable for use in connection with the process.

BACKGROUND OF THE INVENTION

The catalytic epoxidation of olefins using a silver-based catalyst has been known for a long time. Conventional silver-based catalysts have provided the olefin oxides notoriously in a low selectivity. For example, when using conventional catalysts in the epoxidation of ethylene, the selectivity towards ethylene oxide, expressed as a fraction of the ethylene converted, does not reach values above the 6/7 or 85.7 mole-% limit. Therefore, this limit has long been considered to be the theoretically maximal selectivity of this reaction, based on the stoichiometry of the reaction equation

$$7\ C_2H_4 + 6\ O_2 \Rightarrow 6\ C_2H_4O + 2\ CO_2 + 2\ H_2O,$$

cf. Kirk-Othmer's *Encyclopedia* of *Chemical Technology,* 3rd ed., Vol. 9, 1980, p. 445.

Modern silver-based catalysts however are highly selective towards olefin oxide production. When using the modern catalysts in the epoxidation of ethylene the selectivity towards ethylene oxide can reach values above the 6/7 or 85.7 mole-% limit referred to. Such highly selective catalysts, which may comprise as their active components silver, rhenium, at least one further metal and optionally a rhenium co-promoter, are disclosed in US-A-4761394, US-A-4766105, EP-A-266015 and in several subsequent patent publications.

The silver based catalysts are subject to an aging-related performance decline during normal operation and they need to be exchanged periodically. The aging manifests itself by a reduction in the activity of the catalyst. Usually, when a reduction in activity of the catalyst is manifest, the reaction temperature is increased in order to compensate for the reduction in activity. The reaction temperature may be increased until it becomes undesirably high, at which point in time the catalyst is deemed to be at the end of its lifetime and would need to be exchanged.

A reaction modifier, for example an organic halide, may be added to the feed to an epoxidation reactor for increasing the selectivity (cf. for example EP-A-352850). The reaction modifier suppresses the undesirable oxidation of olefin or olefin oxide to carbon dioxide and water, relative to the desired formation of olefin oxide, by a so-far unexplained mechanism.

The optimal quantity of the reaction modifier depends on the epoxidation reaction conditions and on the type of catalyst used. Conventional catalysts have relatively flat selectivity curves for the modifier, i.e. the curves of the selectivity as a function of the quantity of the reaction modifier show that the selectivities are almost invariant over a wide range of reaction modifier quantities, and this property does virtually not change as a function of the reaction temperature and during prolonged operation of the catalyst. Therefore, when using a conventional catalyst, for optimum selectivity the quantity of the reaction modifier can be chosen rather freely and it can remain substantially the same during the entire lifetime of the catalyst.

By contrast, the highly selective catalysts tend to exhibit relatively steep selectivity curves for the modifier, viz. for the highly selective catalysts the selectivity varies considerably with relatively small changes in the quantity of the reaction modifier, and the selectivity exhibits a pronounced maximum, i.e. an optimum, at a certain quantity of the reaction modifier. This has been illustrated in EP-A-352850 (cf. Figure 3 therein). Moreover, the selectivity curves and more in particular this quantity of the reaction modifier where the selectivity is at optimum tend to change with the reaction temperature and, thus, during the catalyst life.

Consequently, when employing the highly selective catalysts in combination with a reaction modifier, the selectivity may vary to an undesirably large extent with changes of the reaction temperature and over the lifetime of the catalyst. Namely, when the reaction temperature is changed, for example to compensate for a reduction in the activity of the catalyst, it represents itself as a problem to maintain reaction conditions which are optimal with respect to the selectivity towards the olefin oxide production.

SUMMARY OF THE INVENTION

It has been found that more of the reaction modifier will generally be needed to achieve a certain effect as the concentration of hydrocarbons in the feed changes to a higher value, and vice versa. It is thought that, unlike other components of the feed, the hydrocarbons present (for example, the olefin and saturated hydrocarbons, if present) have an ability to remove or strip reaction modifier from the catalyst and it is the concentration of the modifier's active species on the catalyst which needs to be maintained, as opposed to the concentration of the reaction modifier at places of the reaction mixture other than the catalyst surface. For this reason, the relative quantity Q of the reaction modifier is considered. The relative quantity Q is basically the ratio of the molar quantity of the reaction modifier to the molar quantity of hydrocarbons as present in the feed. However, as there may be differences in the removing/stripping behavior of the various hydrocarbons in the feed, it may be preferred, when calculating Q, to replace the molar quantity of hydrocarbons by a -so-called-effective molar quantity of hydrocarbons. The effective molar quantity of hydrocarbons in the feed can be calculated from the feed composition (as set out hereinafter), such that it accounts for the differences in the removing/stripping behavior between the hydrocarbons present. There may also be differences in the behavior of different reaction modifiers, while in practice a mixture of reaction modifiers is frequently present. Therefore it may be preferred, when calculating Q, also to replace the molar quantity of the reaction modifier by a -so-called-effective molar quantity of active species of the reaction modifier. The effective molar quantity of active species of the reaction modifier in the feed can be calculated from the feed composition (as set out hereinafter), such that it accounts for the differences in the behavior of different reaction modifiers.

For the highly selective catalysts it has surprisingly been found that when the reaction temperature is increased or decreased the position of the selectivity curve for the modifier shifts towards a higher value of Q or a lower value of Q, respectively, proportionally with the change in the reaction temperature. The proportionality of this shift is independent of the degree of aging of the catalyst and can be determined and verified by routine experimentation.

As a consequence of this finding, when the reaction temperature is changed in the course of the epoxidation process undesirable deviations from the optimum selectivity can be reduced or prevented by adjusting the value of Q proportionally to the change in the reaction temperature. This is particularly useful when the process is operated at optimum conditions with respect to the selectivity, in which case optimum conditions can be maintained by changing the value of Q in proportion to a change in reaction temperature. This all is even more useful when an increase in reaction temperature is employed in response to a reduction in the activity of the catalyst. The invention enables predetermining, for example by calculation, an appropriate change in the value of Q, and therefore in the composition of the reaction modifier and/or the hydrocarbons, in response to a change in the reaction temperature. It is an advantage of this invention that co-currently with the change in reaction temperature there may be changes in the feed composition other than those relating to the hydrocarbons and/or the reaction modifier. Thus, it is a benefit of the present invention that it allows the epoxidation process to be controlled significantly more simply and more smoothly than without the invention.

Accordingly, the present invention provides a process for the epoxidation of an olefin, which process comprises reacting a feed comprising the olefin, oxygen and a reaction modifier in the presence of a highly selective silver-based catalyst at a reaction temperature T, and with the reaction modifier being present in a relative quantity Q which is the ratio of an effective molar quantity of active species of the reaction modifier present in the feed to an effective molar quantity of hydrocarbons present in the feed, and which process comprises the steps of:

- operating at a first operating phase wherein the value of T is $T_1$ and the value of Q is $Q_1$, and
- subsequently operating at a second operating phase at a reaction temperature which is different from the reaction temperature employed in the first operating phase, such that the value of T is $T_2$ and the value of Q is $Q_2$, whereby $Q_2$ is determined by calculation and $Q_2$ is defined by the formula

$$Q_2 = Q_1 + B(T_2 - T_1),$$

wherein B denotes a constant factor which is greater than 0.

The invention also provides a reaction system suitable for performing a process for the epoxidation of an olefin, which reaction system comprises a reactor holding a highly selective silver-based catalyst, being configured to receive a feed comprising the olefin, oxygen and a reaction modifier and having a temperature control system configured to control in the reactor a reaction temperature T, which reaction system further comprises a feed control system configured to control the reaction modifier being present in the feed in a relative quantity Q which is the ratio of an effective molar quantity of active species of the reaction modifier present in the feed to an effective molar quantity of hydrocarbons present in the feed, and which reaction system is configured to perform the process steps of:

- operating at a first operating phase wherein the value of T is $T_1$ and the value of Q is $Q_1$, and
- subsequently operating at a second operating phase at a reaction temperature which is different from the reaction temperature employed in the first operating phase, such that the value of T is $T_2$ and the value of Q is $Q_2$, whereby $Q_2$ can be determined by calculation and $Q_2$ is defined by the formula

$$Q_2 = Q_1 + B(T_2 - T_1),$$

wherein B denotes a constant factor which is greater than 0.

The invention enables to optimally retain the benefits of the reaction modifier while changing the reaction temperature, by maintaining the concentration of the modifier's active species on the catalyst, as opposed to maintaining the concentration of the reaction modifier at places of the reaction mixture other than the catalyst surface. Maintaining the concentration of the modifier's active species on the catalyst is (substantially) achieved when the process is operated in accordance with the formula as defined, in response to a change in reaction temperature. The skilled person is well aware that the formula as defined may be replaced by another mathematical expression, for example a polynomial or exponential expression, which may be constructed such that it provides essentially the same result within the relevant numerical ranges of the variables involved, in particular the ranges as defined hereinafter for values of Q ($Q_1$ and/or $Q_2$) and the reaction temperature T ($T_1$, $T_2$ and/or $T_2 - T_1$). Such embodiments, i.e. which use such an equivalent mathematical expression, fall within the scope of the present invention.

The invention also provides a computer program product which comprises a computer readable medium and a computer readable program code, recorded on the computer readable medium, suitable for instructing a data processing system of a computer system to execute calculations for a process for the epoxidation of an olefin, which process comprises reacting a feed comprising the olefin, oxygen and a reaction modifier in the presence of a highly selective silver-based catalyst at a reaction temperature T, and with the reaction modifier being present in a relative quantity Q which is the ratio of an effective molar quantity of active species of the reaction modifier present in the feed to an effective molar quantity of hydrocarbons present in the feed, and which process comprises the steps of:

- operating at a first operating phase wherein the value of T is $T_1$ and the value of Q is $Q_1$, and
- subsequently operating at a second operating phase at a reaction temperature which is different from the reaction temperature employed in the first operating phase, such that the value of T is $T_2$ and the value of Q is $Q_2$ which is calculated by using the formula

$$Q_2 = Q_1 + B\,(T_2 - T_1).$$

wherein B denotes a constant factor which is greater than 0, or by using another mathematical expression which is constructed such that it provides essentially the same result as the formula.

The invention also provides a computer system which comprises a computer program product and a data processing system configured to receive instructions read from the computer program product, wherein the computer program product comprises a computer readable medium and a computer readable program code, recorded on the computer readable medium, suitable for instructing the data processing system to execute calculations for a process for the epoxidation of an olefin, which process comprises reacting a feed comprising the olefin, oxygen and a reaction modifier in the presence of a highly selective silver-based catalyst at a reaction temperature T, and with the reaction modifier being present in a relative quantity Q which is the ratio of an effective molar quantity of active species of the reaction modifier present in the feed to an effective molar quantity of hydrocarbons present in the feed, and which process comprises the steps of:

- operating at a first operating phase wherein the value of T is $T_1$ and the value of Q is $Q_1$, and
- subsequently operating at a second operating phase at a reaction temperature which is different from the reaction temperature employed in the first operating phase, such that the value of T is $T_2$ and the value of Q is $Q_2$ which is calculated by using the formula

$$Q_2 = Q_1 + B\,(T_2 - T_1),$$

wherein B denotes a constant factor which is greater than 0, or by using another mathematical expression which is constructed such that it provides essentially the same result as the formula.

The present invention also provides, in more general terms, a process for the epoxidation of an olefin, which process comprises reacting a feed comprising the olefin, oxygen and a reaction modifier in the presence of a highly selective silver-based catalyst, and which process comprises the steps of:

- operating at a first operating phase, and
- subsequently operating at a second operating phase at a reaction temperature which is different from the reaction temperature employed in the first operating phase, such that the concentration of active species of the reaction modifier on the catalyst is substantially unchanged.

DETAILED DESCRIPTION OF THE INVENTION

Although the present epoxidation process may be carried out in many ways, it is preferred to carry it out as a gas phase process, i.e. a process in which the feed is contacted in the gas phase with the catalyst which is present as a solid material, typically in a packed bed. Generally the process is carried out as a continuous process. Frequently, in commercial scale operation, the process of the invention may involve a quantity of catalyst which is at least 10 kg, for example at least 20 kg, frequently in the range of from $10^2$ to $10^7$ kg, more frequently in the range of from $10^3$ to $10^6$ kg.

The olefin for use in the present epoxidation process may be any olefin, such as an aromatic olefin, for example styrene, or a di-olefin, whether conjugated or not, for example 1,9-decadiene or 1,3-butadiene. Typically, the olefin is a monoolefin, for example 2-butene or isobutene. Preferably, the olefin is a mono-α-olefin, for example 1-butene or propylene. The most preferred olefin is ethylene.

The olefin concentration in the feed is not material to this invention and may be selected within a wide range. Typically, the olefin concentration in the feed will be at most 80 mole-%, relative to the total feed. Preferably, it will be in the range of from 0.5 to 70 mole-%, in particular from 1 to 60 mole-%, on the same basis. As used herein, the feed is considered to be the composition which is contacted with the catalyst. If desired, the olefin concentration may be increased during the lifetime of the catalyst, by which the selectivity may be improved in an operating phase wherein the catalyst has aged (cf. US-6372925-B1 and WO-A-01/96324, i.e. non-prepublished PCT patent application PCT/US01/18097).

The present epoxidation process may be air-based or oxygen-based, see Kirk-Othmer's *Encyclopedia of Chemical Technology,* 3rd ed., Vol. 9, 1980, p. 445-447. In the air-based process air or air enriched with oxygen is employed as the source of the oxidizing agent while in the oxygen-based processes high-purity (>95 mole-%) oxygen is employed as the source of the oxidizing agent. Presently most epoxidation plants are oxygen-based and this is a preferred embodiment of the present invention.

The oxygen concentration in the feed is not material to this invention and may be selected within a wide range. However, in practice, oxygen is generally applied at a concentration which avoids the flammable regime. Typically, the concentration of oxygen applied will be within the range of from 1 to 15 mole-%, more typically from 2 to 12 mole-% of the total feed.

In order to remain outside the flammable regime, the concentration of oxygen in the feed may be lowered as the concentration of the olefin is increased. The actual safe operating ranges depend, along with the feed composition, also on the reaction conditions such as the reaction temperature and the pressure.

The reaction modifier is present in the feed for increasing the selectivity, suppressing the undesirable oxidation of olefin or olefin oxide to carbon dioxide and water, relative to the desired formation of olefin oxide. Many organic compounds, especially organic halides and organic nitrogen compounds, may be employed as the reaction modifier. Nitrogen oxides, hydrazine, hydroxylamine or ammonia may be employed as well. It is frequently considered that under the operating conditions of olefin epoxidation the nitrogen containing reaction modifiers are precursors of nitrates or nitrites, i.e. they are so-called nitrate- or nitrite-forming compounds (cf. e.g. EP-A-3642, US-A-4822900).

Organic halides are the preferred reaction modifiers, in particular organic bromides, and more in particular organic chlorides. Preferred organic halides are chlorohydrocarbons or bromohydrocarbons. More preferably they are selected from the group of methyl chloride, ethyl chloride, ethylene dichloride, ethylene dibromide, vinyl chloride or a mixture thereof. Most preferred reaction modifiers are ethyl chloride and ethylene dichloride.

Suitable nitrogen oxides are of the general formula $NO_x$ wherein x, which denotes the ratio of the number of oxygen atoms to the number of nitrogen atoms, is in the range of from 1 to 2. These nitrogen oxides include for example NO, $N_2O_3$ and $N_2O_4$. Suitable organic nitrogen compounds are nitro compounds, nitroso compounds, amines, nitrates and nitrites, for example nitromethane, 1-nitropropane or 2-nitropropane. In preferred embodiments, nitrate- or nitrite-forming compounds, e.g. nitrogen oxides and/or organic nitrogen compounds, are used together with an organic halide, in particular an organic chloride.

Although the reaction modifier may be supplied as a single compound, upon contact with the catalyst a variety of compounds may be formed which function as reaction modifier, and which may be present in the feed if a recycle is applied. For example, when applying ethyl chloride in an ethylene oxide process, the feed may in practice comprise ethyl chloride, vinyl chloride, ethylene dichloride and methyl chloride.

The reaction modifiers are generally effective when used in low concentration in the feed, for example up to 0.1 mole-%, relative to the total feed, for example from $0.01 \times 10^{-4}$ to 0.01 mole-%. In particular when the olefin is ethylene, it is preferred that the reaction modifier is present in the feed at a concentration of from $0.05 \times 10^{-4}$ to $50 \times 10^{-4}$ mole-%, in particular from $0.2 \times 10^{-4}$ to $30 \times 10^{-4}$ mole-%, relative to the total feed.

In addition to the olefin, oxygen and the reaction modifier, the feed may contain one or more optional components, such as carbon dioxide, water, inert gases and saturated hydrocarbons. Carbon dioxide and water are a byproducts of the epoxidation process. Carbon dioxide generally has an adverse effect on the catalyst activity. Typically, a concentration of carbon dioxide in the feed in excess of 10 mole-%, preferably in excess of 5 mole-%, relative to the total feed, is avoided. A concentration of carbon dioxide as low as 1 mole-% or lower, relative to the total feed, may be employed. Water may be introduced in the feed as a result of the recovery of olefin oxide and carbon dioxide from the reaction product. Water generally has an adverse effect on the catalyst activity. Typically, a concentration of water in the feed in excess of 3 mole-%, preferably in excess of 1 mole-%, relative to the total feed, is avoided. A concentration of water as low as 0.2 mole-% or lower, relative to the total feed, may be employed. Inert gas, for example nitrogen or argon, or a mixture thereof, may be present in the feed in a concentration of from 0.5 to 95 mole-%. In an air based process inert gas may be present in the feed in a concentration of from 30 to 90 mole-%, typically from 40 to 80 mole-%. In an oxygen based process inert gas may be present in the feed in a concentration of from 0.5 to 30 mole-%, typically from 1 to 15 mole-%. Suitable saturated hydrocarbons are propane and cyclopropane, and in particular methane and ethane. If saturated hydrocarbons are present, they may be present in a quantity of up to 80 mole-%, relative to the total feed, in particular up to 75 mole-%. Frequently they are present in a quantity of at least 30 mole-%, more frequently at least 40 mole-%. Saturated hydrocarbons may be added to the feed in order to increase the oxygen flammability limit.

The relative quantity Q of the reaction modifier is the ratio of the effective molar quantity of active species of the reaction modifier present in the feed to the effective molar quantity of hydrocarbons present in the feed, both molar quantities being expressed in the same units, for example as mole-%, based on the total feed.

When the reaction modifier is a halogen compound, for the purpose of calculating the effective molar quantity of active species of the reaction modifier and the value of Q, the number of active species is deemed to be the number of halogen atoms, and when the reaction modifier is a nitrate- or nitrite-forming compound, the number of active species is deemed to be the number of nitrogen atoms. This implies, for example, that 1 mole of ethylene dichloride provides about 2 moles of active species, i.e. all of the chlorine atoms present provide an active species. On the other hand, it has also been found that reaction modifiers which are methyl compounds, such as methyl chloride and methyl bromide, are less responsive and therefore from 2 to 5 moles, in particular from 2.5 to 3.5 moles, suitably about 3 moles of the methyl compounds may be deemed to provide 1 mole of the active species. This number may be determined and verified by routine experimentation, and -without wishing to be bound by theory- it is believed that this number is higher as the methyl compound in question has a lesser ability to split off the heteroatom in question (for example the halogen or nitrogen atom). Thus, for example, when the feed comprises $2 \times 10^{-4}$ mole-% of ethyl chloride, $3 \times 10^{-4}$ mole-% of vinyl chloride, $1 \times 10^{-4}$ mole-% of ethylene dichloride and $1.5 \times 10^{-4}$ mole-% of methyl chloride, the effective molar quantity of active species of the reaction modifier may be calculated to amount to $2 \times 10^{-4} \times 1 + 3 \times 10^{-4} \times 1 + 1 \times 10^{-4} \times 2 + 1.5 \times 10^{-4} \times 1/3 = 7.5 \times 10^{-4}$ mole-%.

Summarizing, the effective molar quantity of active species of the reaction modifier present in the feed may be calculated by multiplying the molar quantity of each of the reaction modifiers present in the feed with a factor, and adding up the multiplication products, wherein each factor represents the number of active heteroatoms, in particular halogen atoms and/or nitrogen atoms, present per molecule of the reaction modifier in question, on the understanding that the factor for a reaction modifier which is a methyl compound may be in the range of from $1/5$ to $1/2$ more typically from $1/3.5$ to $1/2.5$, suitably about $1/3$.

The hydrocarbons present in the feed comprise the olefin and any saturated hydrocarbon present. As indicated hereinbefore, it is thought that the hydrocarbons present in the feed have the ability to remove/ strip reaction modifier from the catalyst surface and the extent to which they have this ability may differ for the various hydrocarbons. In order to account for these differences (relative to ethylene), the molar quantity of each of the hydrocarbons present is multiplied with a factor, before the molar quantities are added up to calculate the effective molar quantity of the hydrocarbons. Herein, the factor of ethylene is 1, by definition; the factor for methane may be at most 0.5, or at most 0.4, typically in the range of from 0 to 0.2, more typically in the range of from 0 to 0.1; the factor for ethane may be in the range of from 50 to 150, more typically from 70 to 120; and the factor for higher hydrocarbons (i.e. having at least 3 carbon atoms) may be in the range of from 10 to 10000, more typically from 50 to 2000. Such factors may be determined and verified by routine experimentation, and -without wishing to be bound by theory- it is believed that the factor is higher as the hydrocarbon in question has a greater ability to form radicals. Suitable factors for methane, ethane, propane and cyclopropane, relative to ethylene, are about 0.1, about 85, about 1000 and about 60, respectively. As an example, when the feed comprises 30 mole-% ethylene, 40 mole-% of methane, 0.4 mole-% of ethane and 0.0001 mole-% of propane, the effective molar quantity of the hydrocarbons may be calculated to amount to $30 \times 1 + 40 \times 0.1 + 0.4 \times 85 + 0.0001 \times 1000 = 68.1$ mole-%.

It is noted that when ethylene oxide is produced from ethylene without further hydrocarbons being present, the effective molar quantity of the hydrocarbons equals the actual molar quantity, and that the addition of ethane or higher hydrocarbons to an ethylene feed contributes significantly to the effective molar quantity, whereas there is relatively little contribution from any methane added.

Eligible values of Q are at least $1 \times 10^{-6}$, and in particular at least $2 \times 10^{-6}$. Eligible values of Q are at most $100 \times 10^{-6}$, and in particular at most $50 \times 10^{-6}$.

In any operating phase of the process the value of Q may be adjusted so as to achieve an optimal selectivity towards the olefin oxide formation. In practice, the value of Q may be adjusted by adjusting the quantity of the reaction modifier present in the feed, while not changing the hydrocarbon concentrations in the feed.

The present epoxidation process may be carried out using reaction temperatures selected from a wide range. In Preferred embodiments the reaction temperature T is expressed in °C, but other temperatures units are also possible, for example °F. Preferably the reaction temperature T is in the range of from 180 to 340 °C, more preferably in the range of from 190 to 325 °C, in particular in the range of from 200 to 300 °C. The epoxidation process may be carried out such that the reaction temperature is not the same at every catalyst particle. If this is the case, the reaction temperature is deemed to be the weight average temperature of the catalyst particles. On the other hand, when the reaction temperature is not the same at every catalyst particle, still the value of the difference $T_2 - T_1$ may practically be the same for all catalyst particles, and may be more easily determined than the separate values of $T_1$ and $T_2$. The difference $T_2 - T_1$ may be equal to a corresponding difference in coolant temperature.

In accordance with this invention, when the reaction temperature is changed from $T_1$ to $T_2$, the value of Q may be changed from $Q_1$ to $Q_2$, so that deviations from the optimum selectivity which would result from the change in reaction temperature are reduced or even prevented. The value of $Q_2$ is typically a calculated value, calculated on the basis of $T_1$, $T_2$ and $Q_1$. In particular, $Q_2$ can be calculated by using the formula

$$Q_2 = Q_1 + B (T_2 - T_1)$$

(i.e. formula (I)), wherein B denotes a constant factor which is greater than 0. If the reaction temperature T is expressed in °C, B is expressed in $(°C)^{-1}$. In this patent document, all numerical values of B are expressed in $(°C)^{-1}$. The skilled person will be able to convert the numerical values of B expressed in $(°C)^{-1}$ to values expressed in another unit which is consistent with the unit in which the reaction temperature T is expressed. The value of B is not material to this invention. The value of B may eligibly be at least $0.01 \times 10^{-6}$, in particular at least $0.1 \times 10^{-6}$. The value of B may eligibly be at most $1 \times 10^{-6}$, in particular at most $0.5 \times 10^{-6}$. Without wishing to be bound by theory, it is thought that the value of B may be dependent of the composition of the catalyst, in particular the catalytically active metals present, and the nature of the active species of the reaction modifier. Suitable values of B may be determined and verified by routine experimentation. A suitable value of B amounts to about $0.22 \times 10^{-6}$, in particular when used in combination with the numbers and factors employed in the example calculations of the effective molar quantity of active species of the reaction modifier and the effective molar quantity of the hydrocarbons, as given hereinbefore.

It is preferred to operate the epoxidation process at the reaction temperature $T_1$ employing such a value of $Q_1$ that the selectivity towards the olefin oxide formation is optimal. When this is the case, the epoxidation process will continue to operate at an optimum selectivity, but not necessarily the same selectivity, when employing the reaction temperature $T_2$ and the value of $Q_2$ in accordance with formula (I).

The reaction temperature may be changed for many purposes. For example, the reaction temperature may be decreased to slow down the epoxidation reaction when the need thereto arises. Alternatively, the reaction temperature may be increased, to speed up the epoxidation reaction, so that more of the olefin oxide is produced per time unit. Combinations are conceivable, for example a temperature increase may after a period of time be followed by a temperature decrease, and vice versa. Any change in the reaction temperature may be gradual, or step wise, for example in one or more steps of 0.1 to 20 °C, in particular 0.2 to 10 °C, more in particular 0.5 to 5 °C, and any change in the reaction temperature may be accompanied with a change in the value of Q in accordance with this invention. Generally, the change in the value of Q from $Q_1$ to $Q_2$ is made co-currently with the change from $T_1$ to $T_2$.

Preferably, as the catalyst ages, the reaction temperature is changed as to compensate for a reduction in the activity of the catalyst. The activity of the catalyst may be monitored by the production rate of the olefin oxide. In order to compensate for the reduction in the activity of the catalyst, the reaction temperature may be increased gradually or in a plurality of steps, for example in steps of from 0.1 to 20 °C, in particular 0.2 to 10 °C, more in particular 0.5 to 5 °C, with co-current changes in the value of Q, in accordance with formula (I).

In particular in a process for preparing ethylene oxide from ethylene, when a fresh catalyst is used, the reaction temperature is typically in the range of from 180 to 300 °C, more typically from 180 to 280 °C, in particular in the range of from 190 to 270 °C, more in particular from 200 to 260 °C; the value of Q is typically in the range of from $1 \times 10^{-6}$ to $20 \times 10^{-6}$, more typically from $3 \times 10^{-6}$ to $15 \times 10^{-6}$; and the concentration of the reaction modifier in the feed is typically in the range of from $0.2 \times 10^{-4}$ to $10 \times 10^{-4}$ mole-%, preferably from $1 \times 10^{-4}$ to $8 \times 10^{-4}$ mole-%, relative to the total feed. In order to compensate for the reduction in the activity of the catalyst, the reaction temperature may be increased gradually or in a plurality of steps typically to a level in the range of from 230 to 340 °C, more typically from 240 to 325 °C, in particular from 250 to 300 °C. The total increase in the reaction temperature may be in the range of from 10 to 140 °C, more typically from 20 to 100 °C. Typically, the value of Q employed after accomplishing such an increase in reaction temperature may be in the range of from $5 \times 10^{-6}$ to $100 \times 10^{-6}$, more typically from $10 \times 10^{-6}$ to $50 \times 10^{-6}$; and the concentration of the reaction modifier in the feed is increased typically to values in the range of from $1 \times 10^{-4}$ to $40 \times 10^{-4}$ mole-%, preferably from $1.5 \times 10^{-4}$ to $30 \times 10^{-4}$ mole-%, relative to the total feed. Herein, a "fresh catalyst" means a catalyst which, in the course of operation of the epoxidation process, has not reached an age defined by a cumulative olefin oxide production of at least $2 \times 10^6$ mole olefin oxide per m³ of catalyst.

It is an advantage of this invention that any change in the value of Q may be effected by a change in the concentration or composition of the reaction modifier in the feed, or by a change in the concentration or composition of the hydrocarbons in the feed, or a combination of both. Co-currently with these changes there may or may not be changes in the composition of the feed with respect to other components, such as oxygen, carbon dioxide or inert gases.

Generally, the highly selective silver based catalyst is a supported catalyst. The support may be selected from a wide range of inert support materials. Such support materials may be natural or artificial inorganic materials and they include silicon carbide, clays, pumice, zeolites, charcoal and alkaline earth metal carbonates, such as calcium carbonate. Preferred are refractory support materials, such as alumina, magnesia, zirconia and silica. The most preferred support material is α-alumina.

The support material is preferably porous and has preferably a surface area, as measured by the B.E.T. method, of less than 20 $m^2/g$ and in particular from 0.05 to 20 $m^2/g$. More preferably the B.E.T. surface area of the support is in the range of 0.1 to 10, in particular from 0.1 to 3.0 $m^2/g$. As used herein, the B.E.T. surface area is deemed to have been measured by the method as described in Brunauer, Emmet and Teller in *J. Am. Chem. Soc.* 60 (1938) 309-316.

As used herein, a highly selective silver-based catalyst is one which, when operated fresh, can exhibit in the gas phase epoxidation of ethylene a theoretical selectivity at zero oxygen conversion, $S_0$, of at least 6/7 or 85.7 %. More in particular, this theoretical selectivity can be accomplished at a reaction temperature of 260 °C. The value of $S_0$ for a given catalyst is found by operating the catalyst, in particular at a reaction temperature of 260 °C, in a range of gas hourly space velocities, resulting in a range of selectivity values and oxygen conversion values corresponding to the range of gas hourly space velocities employed. The selectivity values found are then extrapolated back to the theoretical selectivity at zero oxygen conversion, $S_0$.

Preferred highly selective silver-based catalysts comprise, in addition to silver, one or more of rhenium, molybdenum, tungsten, a Group IA metal, and a nitrate- or nitrite-forming compound, which may each be present in a quantity of from 0.01 to 500 mmole/kg, calculated as the element (rhenium, molybdenum, tungsten, Group IA metal or nitrogen) on the total catalyst. The nitrate- or nitrite-forming compounds and particular selections of nitrate- or nitrite-forming compound are as defined hereinbefore. Rhenium, molybdenum, tungsten, or the nitrate- or nitrite-forming compound may suitably be provided as an oxyanion, for example, as a perrhenate, molybdate, tungstate or nitrate, in salt or acid form.

Of special preference are the silver-based catalysts which comprise rhenium, in addition to silver. Such catalysts are known from US-A-4761394, US-A-4766105 and EP-A-266015. Broadly, they comprise silver, rhenium or compound thereof, a further metal or compound thereof and optionally a rhenium co-promoter which may be selected from one or more of sulfur, phosphorus, boron, and compounds thereof, on the support material. More specifically the further metal is selected from the group of Group IA metals, Group IIA metals, molybdenum, tungsten, chromium, titanium, hafnium, zirconium, vanadium, thallium, thorium, tantalum, niobium, gallium and germanium and mixtures thereof. Preferably the further metal is selected from the Group IA metals such as lithium, potassium, rubidium and cesium and/or from the Group IIA metals such as calcium and barium. Most preferably it is lithium, potassium and/or cesium. Where possible, rhenium, the further metal or the rhenium co-promoter is provided as an oxyanion, in salt or acid form.

Preferred amounts of the components of these catalysts are, when calculated as the element on the total catalyst:

- silver from 10 to 500 g/kg,
- rhenium from 0.01 to 50 mmole/kg,
- the further metal or metals from 0.1 to 500 mmole/kg each, and, if present,
- the rhenium co-promoter or co-promoters from 0.1 to 30 mmole/kg each.

The present epoxidation process is preferably carried out at a reactor inlet pressure in the range of from 1000 to 4000 kPa. "GHSV" or Gas Hourly Space Velocity is the unit volume of gas at normal temperature and pressure (0 °C, 1 atm, i.e. 101.3 kPa) passing over one unit volume of packed catalyst per hour. Preferably, when the epoxidation process is as a gas phase process involving a packed catalyst bed, the GHSV is in the range of from 1500 to 10000 Nl/(l.h). Preferably, the process of this invention is carried out at a work rate in the range of from 0.5 to 10 kmole olefin oxide produced per $m^3$ of catalyst per hour, in particular 0.7 to 8 kmole olefin oxide produced per $m^3$ of catalyst per hour, for example 5 kmole olefin oxide produced per $m^3$ of catalyst per hour. As used herein, the work rate is the amount of the olefin oxide produced per unit volume of catalyst per hour and the selectivity is the molar quantity of the olefin oxide formed relative to the molar quantity of the olefin converted.

The olefin oxide produced may be recovered from the reaction product by using methods known in the art, for example by absorbing the olefin oxide from a reactor outlet stream in water and optionally recovering the olefin oxide from the aqueous solution by distillation. At least a portion of the aqueous solution containing the olefin oxide may be applied in a subsequent process for converting the olefin oxide into a 1,2-diol or a 1,2-diol ether.

The olefin oxide produced in the present epoxidation process may be converted into a 1,2-diol or into a 1,2-diol ether. As this invention leads to a more attractive process for the production of the olefin oxide, it concurrently leads to a more attractive process which comprises producing the olefin oxide in accordance with the invention and the subsequent use of the obtained olefin oxide in the manufacture of the 1,2-diol and/or 1,2-diol ether.

The conversion into the 1,2-diol or the 1,2-diol ether may comprise, for example, reacting the olefin oxide with water, suitably using an acidic or a basic catalyst. For example, for making predominantly the 1,2-diol and less 1,2-diol ether, the olefin oxide may be reacted with a ten fold molar excess of water, in a liquid phase reaction in presence of an acid catalyst, e.g. 0.5-1.0 %w sulfuric acid, based on the total reaction mixture, at 50-70 °C at 1 bar absolute, or in a gas phase reaction at 130-240 °C and 20-40 bar absolute, preferably in the absence of a catalyst. If the proportion of water is lowered the proportion of 1,2-diol ethers in the reaction mixture is increased. The 1,2-diol ethers thus produced may be a di-ether, tri-ether, tetra-ether or a subsequent ether. Alternative 1,2-diol ethers may be prepared by converting the olefin oxide with an alcohol, in particular a primary alcohol, such as methanol or ethanol, by replacing at least a portion of the water by the alcohol.

The 1,2-diol and the 1,2-diol ether may be used in a large variety of industrial applications, for example in the fields of food, beverages, tobacco, cosmetics, thermoplastic polymers, curable resin systems, detergents, heat transfer systems, etc.

Unless specified otherwise, the organic compounds mentioned herein, for example the olefins, 1,2-diols, 1,2-diol ethers and reaction modifiers, have typically at most 40 carbon atoms, more typically at most 20 carbon atoms, in particular at most 10 carbon atoms, more in particular at most 6 carbon atoms. As defined herein, ranges for numbers of carbon atoms (i.e. carbon number) include the numbers specified for the limits of the ranges.

The reaction system suitable for performing the process of this invention comprises a reactor holding a highly selective silver-based catalyst. The reactor may be configured to receive a feed comprising the olefin, oxygen and the reaction modifier. The reactor may have a temperature control system configured to control in the reactor the reaction temperature T. The reaction system may further comprise a feed control system configured to control the reaction modifier being present in the feed in the relative quantity Q, and optionally the concentration of other feed components.

The computer program product of this invention may comprise a computer readable medium and a computer readable program code, recorded on the computer readable medium. The computer readable program code may be suitable for instructing a data processing system of the computer system of this invention to execute the calculations in connection with the process of this invention. The computer program product may be in the form of a disk which is a permanent entity of the computer system, or it may be a disk which is insertable into the computer system. The computer readable medium may be readable, for example, by means of an optical system or by means of a magnetic system.

In a preferred embodiment, the computer program product may comprise, in addition, a computer readable program code, recorded on the computer readable medium, suitable for instructing the data processing system to control the process of this invention. In particular in this preferred embodiment, the computer system may be configured to communicate with the temperature control system and with the feed control system. This arrangement of configurations facilitates the control of the process steps of the process of this invention.

The following examples will illustrate the invention.

EXAMPLE 1

A catalyst, as defined in US-A-4766105 (EP-A-266015), containing silver, rhenium, cesium, lithium and tungsten on $\alpha$-alumina and having a theoretical selectivity $S_0$ of at least 85.7% in the fresh state was employed in the following experiment.

In the experiment ethylene oxide was produced as follows. A sample of the crushed catalyst (0.8-1.4 mm) was loaded into a micro-reactor consisting of a 3 mm internal diameter stainless steel U-shaped tube. The U-shaped tube was immersed in a molten metal tin/bismuth bath (heat medium) and the ends were connected to a gas flow system. The weight of the catalyst and the inlet gas flow rate were adjusted as to achieve a gas hourly space velocity of 5000 Nl/(l.h). The inlet pressure was 1870 kPa (absolute). The feed to the reactor comprised ethylene at a concentration of 28 mole-%, oxygen at a concentration of 7.4 mole-%, carbon dioxide at a concentration of 5.2 mole-%, ethyl chloride at a concentration of 2.9 ppmv (parts by million by volume), that is such that the selectivity to ethylene oxide is optimal, and the remainder of the feed was nitrogen. The reaction temperature was 257 °C, at which temperature the work rate was 208 kg ethylene oxide/($m^3$.hr). These conditions are hereinafter referred to as "reaction conditions 1".

Subsequently, the reaction conditions 1 were changed to the following: gas hourly space velocity 6000 N1 /(l.h), inlet pressure is 2250 kPa (absolute), ethylene concentration 23 mole-%, oxygen concentration 6.1 mole-%, carbon dioxide concentration of 4.3 mole-%, reaction temperature 263 °C. After optimization of the selectivity to ethylene oxide the ethyl chloride concentration was 2.6 ppmv. The work rate was 251 kg ethylene oxide/($m^3$.hr). These conditions are hereinafter referred to as "reaction conditions 2". By calculation in accordance with formula (I), using B equals $0.22 \times 10^{-6}$ and the reaction conditions 1, it was found that for optimal selectivity to ethylene oxide under the reaction conditions 2 the ethyl chloride concentration should amount to 2.7 ppmv (experimentally found 2.6 ppmv).

Subsequently, the reaction conditions 2 were changed to the following: gas hourly space velocity 7400 Nl /(l.h), inlet pressure is 2650 kPa (absolute), ethylene concentration 19 mole-%, oxygen concentration 5 mole-%, carbon dioxide concentration of 3.5 mole-%, reaction temperature 271 °C. After optimization of the selectivity to ethylene oxide the ethyl chloride concentration was 2.6 ppmv. The work rate was 307 kg ethylene oxide/($m^3$.

hr). These conditions are hereinafter referred to as "reaction conditions 3". By calculation in accordance with formula (I), using B equals $0.22 \times 10^{-6}$ and the reaction conditions 1, it was found that for optimal selectivity to ethylene oxide under the reaction conditions 3 the ethyl chloride concentration should amount to 2.6 ppmv (experimentally found 2.6 ppmv).

EXAMPLE 2 (hypothetical)

A catalyst, as defined in US-A-4766105 (EP-A-266015), containing silver, rhenium, cesium, lithium and sulfur on $\alpha$-alumina and having a theoretical selectivity $S_0$ of 93% in the fresh state is employed in the following experiment. The above value of $S_0$ was determined by measuring selectivities in a range of gas hourly space velocities, each time at 30% ethylene, 8% oxygen, 5% carbon dioxide and 1400 kPa, the reaction temperature being 260 °C, and extrapolating back to zero oxygen conversion.

In the experiment ethylene oxide is produced as follows. A sample of the crushed catalyst (0.8-1.4 mm) is loaded into a micro-reactor consisting of a 3 mm internal diameter stainless steel U-shaped tube. The U-shaped tube is immersed in a molten metal tin/bismuth bath (heat medium) and the ends are connected to a gas flow system. The weight of the catalyst and the inlet gas flow rate are adjusted as to achieve a gas hourly space velocity of 3300 Nl/(l.h). The inlet pressure is 1600 kPa (absolute). The feed to the reactor comprises ethylene at a concentration of 50 mole-%, oxygen at a concentration of 7 mole-%, carbon dioxide at a concentration of 3.5 mole-%, ethyl chloride at a concentration such that the selectivity to ethylene oxide is optimal, and the remainder of the feed is nitrogen. The reaction temperature is 254 °C, at which temperature the work rate is 200 kg ethylene oxide/($m^3$.hr). As the catalyst ages, the reaction temperature is increased gradually such that a constant work rate is maintained. Co-currently with the increase in reaction temperature the concentration of ethyl chloride in the feed is increased in accordance with formula (I), by using B equals $0.22 \times 10^{-6}$.

**Claims**

1.  A process for the epoxidation of an olefin, which process comprises reacting a feed comprising the olefin, oxygen and a reaction modifier in the presence of a highly selective silver-based catalyst at a reaction temperature T, and with the reaction modifier being present in a relative quantity Q which is the ratio of an effective molar quantity of active species of the reaction modifier present in the feed to an effective molar quantity of hydrocarbons present in the feed, and which process comprises the steps of:

- operating at a first operating phase wherein the value of T is $T_1$ and the value of Q is $Q_1$, and
- subsequently operating at a second operating phase at a reaction temperature which is different from the reaction temperature employed in the first operating phase, such that the value of T is $T_2$ and the value of Q is $Q_2$, whereby $Q_2$ is determined by calculation and $Q_2$ is defined by the formula

$$Q_2 = Q_1 + B (T_2 - T_1),$$

wherein B denotes a constant factor which is greater than 0.

2.  A process as claimed in claim 1, wherein the olefin is ethylene, and wherein the reaction modifier comprises an organic chloride.

3.  A process as claimed in claim 2, wherein the reaction modifier comprises chlorohydrocarbons having up to 6 carbon atoms, which comprise one or more of methyl chloride, ethyl chloride, ethylene dichloride and vinyl chloride.

4.  A process as claimed in any of claims 1-3, wherein the effective molar quantity of active species of the reaction modifier present in the feed can be calculated by multiplying the molar quantity of each of the reaction modifiers present in the feed with a factor, and adding up the multiplication products, wherein each factor represents the number of active heteroatoms present per molecule of the reaction modifier in question, on the understanding that the factor for any reaction modifier which is a methyl compound is in the range of from $1/5$ to $1/2$, in particular in the range of from $1/3.5$ to $1/2.5$.

5.  A process as claimed in any of claims 1-4, wherein the effective molar quantity of hydrocarbons present in the feed can be calculated by multiplying the molar quantity of each of the hydrocarbons present in the feed with a factor, and adding up the multiplication products, wherein the factor for any methane is in the range of from 0 to 0.5; the factor for any ethane is in the range of from 50 to 150; and the factor for any hydrocarbon having at least 3 carbon atoms is in the range of from 10 to 10000, all factors being relative to the factor for ethylene, which equals 1, by definition.

6.  A process as claimed in claim 5, wherein the factor for any methane is in the range of from 0 to 0.4; the factor for any ethane is in the range of from 70 to 120; and the factor for any hydrocarbon having at least 3 carbon atoms is in the range of from 50 to 2000, all factors being relative to the factor for ethylene, which equals 1, by definition.

7. A process as claimed in any of claims 1-6, wherein the relative quantity Q is in the range of from $1 \times 10^{-6}$ to $100 \times 10^{-6}$, in particular in the range of from $2 \times 10^{-6}$ to $50 \times 10^{-6}$.

8. A process as claimed in any of claims 1-7, wherein the reaction temperature T is in the range of from 190 to 325 °C, in particular in the range of from 200 to 300 °C.

9. A process as claimed in any of claims 1-8, wherein the reaction temperature T is expressed in °C and the value of B, expressed in $(°C)^{-1}$, is in the range of from $0.01 \times 10^{-6}$ to $1 \times 10^{-6}$, in particular in the range of from $0.1 \times 10^{-6}$ to $0.5 \times 10^{-6}$.

10. A process as claimed in any of claims 1-9, wherein at the reaction temperature $T_1$ such a value of $Q_1$ is employed that the selectivity towards the olefin oxide formation is optimal.

11. A process as claimed in any of claims 1-10, wherein as the catalyst ages, the reaction temperature is changed as to compensate for a reduction in the activity of the catalyst.

12. A process according to any of claims 1-11, wherein the highly selective silver based catalyst comprises one or more of rhenium, molybdenum, tungsten, and a nitrate- or nitrite-forming compound, on a support, in particular an $\alpha$-alumina support.

13. A process according to claim 12, wherein the highly selective silver based catalyst comprises silver, rhenium or compound thereof, a further metal or compound thereof selected from Group IA metals, Group IIA metals, molybdenum, tungsten, chromium, titanium, hafnium, zirconium, vanadium, thallium, thorium, tantalum, niobium, gallium and germanium and mixtures thereof and optionally a rhenium co-promoter selected from one or more of sulfur, phosphorus, boron, and compounds thereof.

14. A process as claimed in any of claims 1-13, wherein the hydrocarbons present in the feed comprise one or more of methane, ethane, propane and cyclopropane, in addition to the olefin.

15. A process as claimed in any of claims 1-14, wherein $Q_2$ is calculated by using the formula

$$Q_2 = Q_1 + B (T_2 - T_1),$$

wherein B denotes a constant factor which is greater than 0, or by using an equivalent mathematical expression.

16. A process for the epoxidation of an olefin, which process comprises reacting a feed comprising the olefin, oxygen and a reaction modifier in the presence of a highly selective silver-based catalyst, and which process comprises the steps of:

 - operating at a first operating phase, and
 - subsequently operating at a second operating phase at a reaction temperature which is different from the reaction temperature employed in the first operating phase, such that the concentration of active species of the reaction modifier on the catalyst is substantially unchanged.

17. A method of using an olefin oxide for making a 1,2-diol or a 1,2-diol ether comprising converting the olefin oxide into the 1,2-diol or the 1,2-diol ether, wherein the olefin oxide has been obtained by a process for the production of olefin oxide according to any of claims 1-16.

18. A reaction system suitable for performing a process for the epoxidation of an olefin, which reaction system comprises a reactor holding a highly selective silver-based catalyst, being configured to receive a feed comprising the olefin, oxygen and a reaction modifier and having a temperature control system configured to control in the reactor a reaction temperature T, which reaction system further comprises a feed control system configured to control the reaction modifier being present in the feed in a relative quantity Q which is the ratio of an effective molar quantity of active species of the reaction modifier present in the feed to an effective molar quantity of hydrocarbons present in the feed, and which reaction system is configured to perform the process steps of:

 - operating at a first operating phase wherein the value of T is $T_1$ and the value of Q is $Q_1$, and
 - subsequently operating at a second operating phase at a reaction temperature which is different from the reaction temperature employed in the first operating phase, such that the value of T is $T_2$ and the value of Q is $Q_2$, whereby $Q_2$ can be determined by calculation and $Q_2$ is defined by the formula

$$Q_2 = Q_1 + B (T_2 - T_1),$$

wherein B denotes a constant factor which is greater than 0.

19. A computer program product which comprises a computer readable medium and a computer readable program code, recorded on the computer readable medium, suitable for instructing a data processing system of a computer system to execute calculations for a process for the epoxidation of an olefin as claimed in any of claims 1-15.

20. A computer program product as claimed in claim 19, which comprises, in addition, a computer readable program code, recorded on the computer readable medium, suitable for instructing the data processing system to control the process for the epoxidation of an olefin.

21. A computer system which comprises a computer program product and a data processing system configured to receive instructions read from the computer program product, wherein the computer program product comprises a computer readable medium and a computer readable program code, recorded on the computer readable medium, suitable for instructing the data processing system to execute calculations for a process for the epoxidation of an olefin as claims in any of claims 1-15.

22. A computer system as claimed in claim 21, wherein the computer system is configured to communicate with a temperature control system configured to control the reaction temperature T and with a feed control system configured to control the reaction modifier being present in the feed in the relative quantity Q, and wherein the computer program product comprises, in addition, a computer readable program code, recorded on the computer readable medium, suitable for instructing the data processing system to control the said process for the epoxidation of an olefin.

**Patentansprüche**

1. Verfahren zur Epoxidierung eines Olefins, welches Verfahren das Umsetzen eines Einsatzmaterials, das das Olefin, Sauerstoff und ein Reaktionsmodifikationsmittel umfaßt, in Gegenwart eines hochselektiven, auf Silber basierenden Katalysators bei einer Reaktionstemperatur T umfaßt, wobei das Reaktionsmodifikationsmittel in einer relativen Menge Q vorhanden ist, welche das Verhältnis einer effektiven molaren Menge an aktiver Spezies des im Einsatzmaterial vorhandenen Reaktionsmodifikationsmittels zu einer effektiven molaren Menge an im Einsatzmaterial vorhandenen Kohlenwasserstoffen ist, und welches Verfahren die Schritte von:

   - Durchführen einer ersten Betriebsphase, worin der Wert von T $T_1$ ist und der Wert von Q $Q_1$ ist, und
   - darauffolgendes Durchführen einer zweiten Betriebsphase bei einer Reaktionstemperatur, welche von der in der ersten Betriebsphase angewandten Reaktionstemperatur verschieden ist, sodaß der Wert von T $T_2$ ist und der Wert von Q $Q_2$ ist, wobei $Q_2$ durch Berechnung ermittelt wird und $Q_2$ durch die Formel

$$Q_2 = Q_1 + B\,(T_2 - T_1)$$

definiert ist, worin B einen konstanten Faktor größer als 0 bezeichnet.

2. Verfahren nach Anspruch 1, worin das Olefin Ethylen ist und worin das Reaktionsmodifikationsmittel ein organisches Chlorid umfaßt.

3. Verfahren nach Anspruch 2, worin das Reaktionsmodifikationsmittel Chlorkohlenwasserstoffe mit bis zu 6 Kohlenstoffatomen umfaßt, welche einen oder mehrere von Methylchlorid, Ethylchlorid, Ethylenchlorid und Vinylchlorid umfassen.

4. Verfahren nach einem der Ansprüche 1 bis 3, worin die effektive molare Menge an aktiven Spezies des im Einsatzmaterial vorhandenen Reaktionsmodifikationsmittels durch Multiplizieren der molaren Menge von jedem der im Einsatzmaterial vorhandenen Reaktionsmodifikationsmittel mit einem Faktor, und Addieren der resultierenden Multiplikationsprodukte berechnet werden kann, wobei jeder Faktor die Anzahl an aktiven Heteroatomen darstellt, welche pro Molekül des in Frage kommenden Reaktionsmodifikationsmittels vorhanden sind, unter der Voraussetzung, daß der Faktor für jedwedes Reaktionsmodifikationsmittel, welches eine Methylverbindung ist, im Bereich von 1/5 bis 1/2, insbesondere im Bereich von 1/3,5 bis 1/2,5 liegt.

5. Verfahren nach einem der Ansprüche 1 bis 4, worin die effektive molare Menge an im Einsatzmaterial vorhandenen Kohlenwasserstoffen durch Multiplizieren der molaren Menge von jedem der im Einsatzmaterial vorhandenen Kohlenwasserstoffe mit einem Faktor, und Addieren der erhaltenen Multiplikationsprodukte berechnet werden kann, wobei der Faktor für jedwedes Methan im Bereich von 0,1 bis 0,5 liegt; der Faktor für jedwedes Ethan im Bereich von 50 bis 150 liegt, und der Faktor für jedwede Kohlenwasserstoffe mit wenigstens 3 Kohlenstoffatomen im Bereich von 10 bis 10.000 liegt, wobei alle Faktoren relativ zum Faktor für Ethylen sind, welcher per Definition 1 entspricht.

6. Verfahren nach Anspruch 5, worin der Faktor für jedwedes Methan im Bereich von 0 bis 0,4 liegt, der Faktor für jedwedes Ethan im Bereich von 70 bis 120 liegt, und der Faktor für jedweden Kohlenwasserstoff mit wenigstens 3 Kohlenstoffatomen im Bereich von 50 bis 2.000 liegt, wobei alle Faktoren relativ zum Faktor für Ethylen sind, welcher per Definition 1 entspricht.

**7.** Verfahren nach einem der Ansprüche 1 bis 6, worin die relative Menge Q im Bereich von $1 \times 10^{-6}$ bis $100 \times 10^{-6}$, insbesondere im Bereich von $2 \times 10^{-6}$ bis $50 \times 10^{-6}$ liegt.

**8.** Verfahren nach einem der Ansprüche 1 bis 7, worin die Reaktionstemperatur T im Bereich von 190 bis 325°C, insbesondere im Bereich von 200 bis 300°C beträgt.

**9.** Verfahren nach einem der Ansprüche 1 bis 8, worin die Reaktionstemperatur T in °C ausgedrückt ist und der Wert von B, ausgedrückt in $(°C)^{-1}$, im Bereich von $0,01 \times 10^{-6}$ bis $1 \times 10^{-6}$, insbesondere im Bereich von $0,1 \times 10^{-6}$ bis $0,5 \times 10^{-6}$ liegt.

**10.** Verfahren nach einem der Ansprüche 1 bis 9, worin bei der Reaktionstemperatur $T_1$ ein derartiger Wert von $Q_1$ angewandt wird, daß die Selektivität hin zur Olefinoxidbildung optimal ist.

**11.** Verfahren nach einem der Ansprüche 1 bis 10, worin mit der Alterung des Katalysators die Reaktionstemperatur verändert wird, um eine Verringerung in der Aktivität des Katalysators zu kompensieren.

**12.** Verfahren nach einem der Ansprüche 1 bis 11, worin der hochselektive, auf Silber basierende Katalysator eines oder mehrere von Rhenium, Molybdän, Wolfram und einer Nitrat- oder Nitrit ausbildenden Verbindung auf einem Träger, insbesondere einem alpha-Aluminiumoxid-Träger umfaßt.

**13.** Verfahren nach Anspruch 12, worin der hochselektive, auf Silber basierende Katalysator Silber, Rhenium oder eine Verbindung hievon, ein weiteres Metall oder eine Verbindung hievon, ausgewählt von Gruppe IA-Metallen, Gruppe IIA-Metallen, Molybdän, Wolfram, Chrom, Titan, Hafnium, Zirconium, Vanadium, Thallium, Thorium, Tantal, Niob, Gallium und Germanium und Gemischen hievon und wahlweise einen Rhenium-Copromotor, ausgewählt unter einem oder mehreren von Schwefel, Phosphor, Bor und Verbindungen hievon umfaßt.

**14.** Verfahren nach einem der Ansprüche 1 bis 13, worin die im Einsatzmaterial vorhandenen Kohlenwasserstoffe zusätzlich zum Olefin einen oder mehrere von Methan, Ethan, Propan und Cyclopropan umfassen.

**15.** Verfahren nach einem der Ansprüche 1 bis 14, worin $Q_2$ durch Verwendung der Formel

$$Q_2 = Q_1 + B (T_2 - T_1),$$

worin B einen konstanten Faktor darstellt, welcher größer als 0 ist, oder unter Verwendung einer äquivalenten mathematischen Formel berechnet wird.

**16.** Verfahren zur Epoxidierung eines Olefins, welches Verfahren das Umsetzen eines das Olefin, Sauerstoff und ein Reaktionsmodifikationsmittel umfassenden Einsatzmaterials in Gegenwart eines hochselektiven, auf Silber basierenden Katalysators umfaßt, welches Verfahren die Schritte von:

- Durchführen einer ersten Betriebsphase, und
- darauffolgendes Durchführen einer zweiten Betriebsphase bei einer Reaktionstemperatur, welche von der in der ersten Betriebsphase angewandten Reaktionstemperatur verschieden ist, umfaßt, sodaß die Konzentration an aktiven Spezies des Reaktionsmodifikationsmittels auf dem Katalysator im wesentlichen unverändert ist.

**17.** Verfahren der Verwendung eines Olefinoxids zur Herstellung eines 1,2-Diols oder eines 1,2-Diolethers, umfassend das Umwandeln eines Olefinoxids in den 1,2-Diol oder den 1,2-Diolether, wobei das Olefinoxid durch ein Verfahren zur Herstellung eines Olefinoxids nach einem der Ansprüche 1 bis 16 erhalten wurde.

**18.** Reaktionssystem, welches zur Durchführung eines Verfahrens zur Epoxidierung eines Olefins geeignet ist, welches Reaktionssystem einen Reaktor, der einen hochselektiven, auf Silber basierenden Katalysator enthält, welcher Reaktor konfiguriert ist, um ein Einsatzmaterial, das das Olefin, Sauerstoff und ein Reaktionsmodifikationsmittel umfaßt, aufzunehmen, und ein Temperatursteuerungssystem aufweist, welches konfiguriert ist, um in dem Reaktor eine Reaktionstemperatur T zu regulieren, welches Reaktionssystem ferner ein Einsatzmaterialsteuerungssystem umfaßt, welches konfiguriert ist, um das im Einsatzmaterial in einer relativen Menge Q vorhandene Reaktionsmodifikationsmittel zu regulieren, wobei Q das Verhältnis aus einer effektiven molaren Menge an aktiven Spezies des im Einsatzmaterial vorhandenen Reaktionsmodifikationsmittels zu einer effektiven molaren Menge an im Einsatzmaterial vorhandenen Kohlenwasserstoffen darstellt, und welches Reaktionssystem konfiguriert ist, das die Verfahrensschritte:

- Durchführen einer ersten Betriebsphase, worin der Wert von T $T_1$ ist, und der Wert von Q $Q_1$ ist, und
- darauffolgendes Durchführen einer zweiten Betriebsphase bei einer Reaktionstemperatur, welche von der in der ersten Betriebsphase angewandten Reaktionstemperatur verschieden ist, sodaß der Wert von T $T_2$ ist und der Wert von Q $Q_2$ ist, wobei $Q_2$ durch Berechnung ermittelt werden kann und $Q_2$ durch die Formel

$$Q_2 = Q_1 + B (T_2 - T_1)$$

definiert ist, worin B einen konstanten Faktor größer als 0 bezeichnet, umfaßt.

19. Computerprogrammprodukt, welches ein computerlesbares Medium und einen computerlesbaren Programmcode umfaßt, welcher auf dem computerlesbaren Medium aufgezeichnet ist, welches zur Steuerung eines Datenverarbeitungssystems eines Computersystems geeignet ist, um Berechnungen für das Verfahren zur Epoxidierung eines Olefins nach einem der Ansprüche 1 bis 15 auszuführen.

20. Computerprogrammprodukt nach Anspruch 19, welches zusätzlich einen computerlesbaren Programmcode, der auf einem computerlesbaren Medium aufgezeichnet ist, umfaßt, der zur Steuerung des Datenverarbeitungssystems zur Überwachung/Steuerung des Verfahrens zur Epoxidierung eines Olefins geeignet ist.

21. Computersystem, welches ein Computerprogrammprodukt und ein Datenverarbeitungssystem umfaßt, welches konfiguriert ist, um vom Computerprogrammprodukt abgelesene Instruktionen zu erhalten, wobei das Computerprogrammprodukt ein computerlesbares Medium und einen computerlesbaren Programmcode umfaßt, welcher auf dem computerlesbaren Medium aufgezeichnet ist, das zur Steuerung eines Datenverarbeitungssystems geeignet ist, um Berechnungen für ein Verfahren zur Epoxidierung eines Olefins nach einem der Ansprüche 1 bis 15 auszuführen.

22. Computersystem nach Anspruch 21, worin das Computersystem konfiguriert ist, um mit einem Temperatursteuerungssystem zu kommunizieren, welches konfiguriert ist, um die Reaktionstemperatur T zu steuern, und mit einem Einsatzmaterialsteuerungssystem, welches konfiguriert ist, um das im Einsatzmaterial in der relativen Menge Q vorhandene Reaktionsmodifikationsmittel zu steuern, und worin das Computerprogrammprodukt zusätzlich einen computerlesbaren Programmcode umfaßt, welcher auf einem computerlesbaren Medium aufgezeichnet ist, der zur Steuerung des Datenverarbeitungssystems zur Überwachung/Steuerung des Verfahrens zur Epoxidierung eines Olefins geeignet ist.

**Revendications**

1. Procédé d'époxydation d'une oléfine, qui comprend la réaction d'une charge d'alimentation comprenant l'oléfine, de l'oxygène et un modificateur de réaction en présence d'un catalyseur à base d'argent hautement sélectif à une température réactionnelle T et avec le modificateur de réaction présent en quantité relative Q, qui est le rapport d'une quantité molaire efficace d'espèces actives du modificateur de réaction présent dans la charge d'alimentation à une quantité molaire efficace d'hydrocarbures présents dans la charge d'alimentation et lequel procédé comprend les étapes suivantes :

on opère dans une première phase de fonctionnement, dans laquelle la valeur de T est $T_1$ et la valeur de Q est $Q_1$, et
on opère ensuite dans une seconde phase de fonctionnement à une température réactionnelle qui est différente de la température réactionnelle employée dans la première phase de fonctionnement et qui est telle que la valeur de T soit $T_2$ et que la valeur de Q soit $Q_2$, $Q_2$ étant déterminée par calcul et $Q_2$ étant définie par la formule :

$$Q_2 = Q_1 + B (T_2 - T_1)$$

dans laquelle B représente un facteur constant qui est supérieur à 0.

2. Procédé selon la revendication 1, dans lequel l'oléfine est l'éthylène et dans lequel le modificateur de réaction comprend un chlorure organique.

3. Procédé selon la revendication 2, dans lequel le modificateur de réaction comprend des chlorohydrocarbures ayant jusqu'à 6 atomes de carbone, qui comprennent un ou plusieurs des composés choisis parmi le chlorure de méthyle, le chlorure d'éthyle, le dichlorure d'éthylène et le chlorure de vinyle.

4. Procédé selon l'une quelconque des revendications 1 à 3, dans lequel la quantité molaire efficace d'espèces actives du modificateur de réaction présent dans la charge d'alimentation peut être calculée en multipliant la quantité molaire de chacun des modificateurs de réaction présents dans la charge d'alimentation par un facteur et en ajoutant les produits de multiplication, chaque facteur représentant le nombre d'hétéroatomes actifs présents par molécule du modificateur de réaction en question, à condition que le facteur de tout modificateur de réaction qui est un composé de méthyle se situe dans la plage de 1/5 à 1/2, en particulier dans la plage de 1/3,5 à 1/2,5.

**5.** Procédé selon l'une quelconque des revendications 1 à 4, dans lequel la quantité molaire efficace d'hydrocarbures présents dans la charge d'alimentation peut être calculée en multipliant la quantité molaire de chacun des hydrocarbures présents dans la charge d'alimentation par un facteur et en ajoutant les produits de multiplication, dans lequel le facteur pour le méthane éventuel se situe dans la plage de 0 à 0,5; le facteur pour l'éthane éventuel se situe dans la plage de 50 à 150; et le facteur pour un hydrocarbure quelconque ayant au moins 3 atomes de carbone se situe dans la plage de 10 à 10.000, tous les facteurs se rapportant au facteur pour l'éthylène qui est égal à 1 par définition.

**6.** Procédé selon la revendication 5, dans lequel le facteur pour le méthane éventuel se situe dans la plage de 0 à 0,4; le facteur pour l'éthane éventuel se situe dans la plage de 70 à 120; et le facteur pour un hydrocarbure quelconque ayant au moins 3 atomes de carbone se situe dans la plage de 50 à 2000, tous les facteurs se rapportant au facteur pour l'éthylène qui est égal à 1 par définition.

**7.** Procédé selon l'une quelconque des revendications 1 à 6, dans lequel la quantité relative Q se situe dans la plage de $1 \times 10^{-6}$ à $100 \times 10^{-6}$, en particulier dans la plage de $2 \times 10^{-6}$ à $50 \times 10^{-6}$.

**8.** Procédé selon l'une quelconque des revendications 1 à 7, dans lequel la température réactionnelle T se situe dans la plage de 190 à 325°C, en particulier dans la plage de 200 à 300°C.

**9.** Procédé selon l'une quelconque des revendications 1 à 8, dans lequel la température réactionnelle T est exprimée en °C et la valeur de B, exprimée en $(°C)^{-1}$, se situe dans la plage de $0,01 \times 10^{-6}$ à $1 \times 10^{-6}$, en particulier dans la plage de $0,1 \times 10^{-6}$ à $5 \times 10^{-6}$.

**10.** Procédé selon l'une quelconque des revendications 1 à 9, dans lequel, à la température réactionnelle $T_1$, cette valeur de $Q_1$ est employée de sorte que la sélectivité pour la formation d'oxyde d'oléfine soit optimale.

**11.** Procédé selon l'une quelconque des revendications 1 à 10, dans lequel, à mesure que le catalyseur vieillit, la température réactionnelle est modifiée de manière à compenser une réduction de l'activité du catalyseur.

**12.** Procédé selon l'une quelconque des revendications 1 à 11, dans lequel le catalyseur à base d'argent hautement sélectif comprend un ou plusieurs des corps suivants : le rhénium, le molybdène, le tungstène et un composé formateur de nitrate ou de nitrite, sur un support, en particulier un support de a-alumine.

**13.** Procédé selon. la revendication 12, dans lequel le catalyseur à base d'argent hautement sélectif comprend de l'argent, du rhénium ou un de leurs composés, un autre métal ou un de ses composés choisi parmi les métaux du groupe IA, les métaux du groupe IIA, le molybdène, le tungstène, le chrome, le titane, l'hafnium, le zirconium, le vanadium, le thallium, le thorium, le tantale, le niobium, le gallium, le germanium et leurs mélanges et, éventuellement, un co-promoteur de rhénium choisi dans le groupe constitué du soufre, du phosphore, du bore et de leurs composés.

**14.** Procédé selon l'une quelconque des revendications 1 à 13, dans lequel les hydrocarbures présents dans la charge d'alimentation comprennent un ou plusieurs hydrocarbures choisis parmi le méthane, l'éthane, le propane et le cyclopropane, en plus de l'oléfine.

**15.** Procédé selon l'une quelconque des revendications 1 à 14, dans lequel $Q_2$ est calculée en utilisant la formule :

$$Q_2 = Q_1 + B (T_2 - T_1),$$

dans laquelle B représente un facteur constant qui est supérieur à 0, ou en utilisant une expression mathématique équivalente.

**16.** Procédé d'époxydation d'une oléfine, qui comprend la réaction d'une charge d'alimentation comprenant l'oléfine, de l'oxygène et un modificateur de réaction en présence d'un catalyseur à base d'argent hautement sélectif, lequel procédé comprend les étapes suivantes :

on opère dans une première phase de fonctionnement et on opère ensuite dans une seconde phase de fonctionnement à une température réactionnelle qui est différente de la température réactionnelle employée dans la première phase de fonctionnement et est telle que la concentration en espèces actives du modificateur de réaction sur le catalyseur soit sensiblement inchangée.

**17.** Procédé d'utilisation d'un oxyde d'oléfine pour la production d'un 1,2-diol ou un éther de 1,2-diol, comprenant la conversion de l'oxyde d'oléfine en 1,2-diol ou en éther de 1,2-diol, dans lequel l'oxyde d'oléfine a été obtenu par un procédé de production d'un oxyde d'oléfine selon l'une quelconque des revendications 1 à 16.

**18.** Système de réaction convenant à la réalisation d'un procédé d'époxydation d'une oléfine, lequel système de réaction comprend un réacteur contenant un catalyseur à base d'argent hautement sélectif et qui est configuré pour recevoir une charge d'alimentation comprenant l'oléfine, de l'oxygène et un modificateur de réaction et ayant un système de réglage de la température configuré pour régler dans le réacteur une température réactionnelle T, lequel système de réaction comprend en outre un système de réglage de charge d'alimentation configuré pour régler le modificateur de réaction présent dans la charge d'alimentation en quantité relative Q, qui est le rapport d'une quantité molaire efficace d'espèces actives du modificateur de réaction présent dans la charge d'alimentation à une quantité molaire efficace d'hydrocarbures présents dans la charge d'alimentation, et lequel système de réaction est configuré pour réaliser les étapes de procédé suivantes :

- on opère dans une première phase de fonctionnement, dans laquelle la valeur de T est $T_1$ et la valeur de Q est $Q_1$, et .
- on opère dans une seconde phase de fonctionnement à une température réactionnelle qui est différente de la température réactionnelle employée dans la première phase de fonctionnement et qui est telle que la valeur de T soit $T_2$ et que la valeur de Q soit $Q_2$, $Q_2$ étant déterminée par calcul et $Q_2$ étant définie par la formule :

$$Q_2 = Q_1 + B (T_2 - T_1),$$

dans laquelle B représente un facteur constant qui est supérieur à 0.

**19.** Produit programme informatique, qui comprend un support lisible par ordinateur et un code de programme lisible par ordinateur, enregistré sur le support lisible par ordinateur, convenant pour instruire un système de traitement de données d'un système informatique d'effectuer des calculs pour un procédé d'époxydation d'une oléfine selon l'une quelconque des revendications 1 à 15.

**20.** Produit programme informatique selon la revendication 19, qui comprend, en outre, un code de programme lisible par ordinateur enregistré sur le support lisible par ordinateur, convenant pour instruire le système de traitement de données de commander le procédé d'époxydation d'une oléfine.

**21.** Système informatique, qui comprend un produit programme informatique et un système de traitement de données configuré pour recevoir des instructions lues dans le produit programmé informatique, lequel produit programme informatique comprend un support lisible par ordinateur et un code de programme lisible par ordinateur, enregistré sur le support lisible par ordinateur, convenant pour instruire le système de traitement de données d'effectuer des calculs pour un procédé d'époxydation d'une oléfine selon l'une quelconque des revendications 1 à 15.

**22.** Système informatique selon la revendication 21, dans lequel le système informatique est configuré pour communiquer avec un système de réglage de la température configuré pour régler la température réactionnelle T et avec un système de réglage de charge d'alimentation configuré pour régler le modificateur de réaction présent dans la charge d'alimentation en quantité relative Q, et dans lequel le produit programme informatique comprend, en outre, un code de programme lisible par ordinateur, enregistré sur le support lisible par ordinateur, convenant pour instruire le système de traitement de données de régler ledit procédé d'époxydation d'une oléfine.